(19)

**Europäisches Patentamt**
**European Patent Office**
**Office européen des brevets**

(11) **EP 4 298 432 B1**

(12) **EUROPEAN PATENT SPECIFICATION**

(45) Date of publication and mention
of the grant of the patent:
**18.09.2024  Bulletin 2024/38**

(51) International Patent Classification (IPC):
**G01N 22/00** *(2006.01)*    **G01N 27/22** *(2006.01)*
**G01N 33/483** *(2006.01)*

(21) Application number: **22705800.5**

(22) Date of filing: **15.02.2022**

(52) Cooperative Patent Classification (CPC):
**G01N 22/00; G01N 27/221; G01N 33/483**

(86) International application number:
**PCT/EP2022/053659**

(87) International publication number:
**WO 2022/179887 (01.09.2022 Gazette 2022/35)**

(54) **A METHOD AND A SYSTEM FOR DETECTING MICROWAVE SIGNALS WHICH CARRY INFORMATION OF THE FUNCTIONAL DYNAMICS OF BIOLOGICAL PARTICLES**

VERFAHREN UND SYSTEM ZUR DETEKTION VON MIKROWELLENSIGNALEN, DIE INFORMATIONEN ÜBER DIE FUNKTIONALE DYNAMIK VON BIOLOGISCHEN TEILCHEN TRAGEN

PROCÉDÉ ET SYSTÈME DE DÉTECTION DE SIGNAUX MICRO-ONDES PORTANT DES INFORMATIONS DE LA DYNAMIQUE FONCTIONNELLE DES PARTICULES BIOLOGIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.02.2021  EP 21382142**

(43) Date of publication of application:
**03.01.2024  Bulletin 2024/01**

(73) Proprietor: **Universitat Politècnica De Catalunya**
**08034 Barcelona (ES)**

(72) Inventors:
  • **JOFRE CRUANYES, Marc**
    **08015 Barcelona (ES)**
  • **JOFRE CRUANYES, Lluís**
    **08360 Barcelona (ES)**
  • **JOFRE ROCA, Luis**
    **08360 Barcelona (ES)**
  • **PALACIOS ARIAS, César Augusto**
    **08520 Barcelona (ES)**
  • **ROMEU ROBERT, Jordi**
    **08173 Barcelona (ES)**
  • **O'CALLAGHAN CASTELLÀ, Juan Manuel**
    **08029 Barcelona (ES)**

(74) Representative: **Torner, Juncosa I Associats, SL**
**C / Pau Claris, 108, 1r 1a**
**08009 Barcelona (ES)**

(56) References cited:
**US-A- 5 144 224          US-A1- 2016 320 316**
**US-A1- 2020 209 169**

  • **BALZANO QUIRINO: "Proposed test for detection of nonlinear responses in biological preparations exposed to RF energy", BIOELECTROMAGNETICS, vol. 23, no. 4, May 2002 (2002-05-01), US, pages 278 - 287, XP055825126, ISSN: 0197-8462, DOI: 10.1002/bem.10017**
  • **BALZANO QUIRINO: "RF nonlinear interactions in living cells-II: Detection methods for spectral signatures", BIOELECTROMAGNETICS, vol. 24, no. 7, October 2003 (2003-10-01), US, pages 483 - 488, XP055825133, ISSN: 0197-8462, DOI: 10.1002/bem.10125**
  • **BALZANO QUIRINO ET AL: "A doubly resonant cavity for detection of RF demodulation by living cells", BIOELECTROMAGNETICS, vol. 29, no. 2, February 2008 (2008-02-01), US, pages 81 - 91, XP055825132, ISSN: 0197-8462, DOI: 10.1002/bem.20365**

EP 4 298 432 B1

- **BALZANO QUIRINO ET AL: "RF nonlinear interactions in living cells-I: Nonequilibrium thermodynamic theory", BIOELECTROMAGNETICS, vol. 24, no. 7, October 2003 (2003-10-01), US, pages 473 - 482, XP055825129, ISSN: 0197-8462, DOI: 10.1002/bem.10126**
- **JEROME J W ET AL: "Computational Modeling Evidence of a Nonthermal Electromagnetic Interaction Mechanism With Living Cells: Microwave Nonlinearity in the Cellular Sodium Ion Channel", IEEE TRANSACTIONS ON MICROWAVE THEORY AND TECHNIQUES, PLENUM, USA, vol. 52, no. 8, August 2004 (2004-08-01), pages 2040 - 2045, XP011115663, ISSN: 0018-9480, DOI: 10.1109/TMTT.2004.831924**
- **BO YANG ET AL: "Non-Invasive Imaging Method of Microwave Near Field Based on Solid State Quantum Sensing", ARXIV.ORG, CORNELL UNIVERSITY LIBRARY, 201 OLIN LIBRARY CORNELL UNIVERSITY ITHACA, NY 14853, 5 January 2018 (2018-01-05), XP081204011, DOI: 10.1109/TMTT.2018.2812204**

## Description

Technical Field

The present invention relates to measurements of the functional dynamics of biological particles. In particular, the invention relates to the measurement of the membrane potential of biological particles with microwave frequency mixing adapted to the characteristic signal signature of the membrane making use of the nonlinearity of the voltage-current response of the membrane of biological particles.

Background of the invention

In neurons (eukaryotic cells) the action potential is used in order to transmit information to other neurons, whilst in bacteria biofilms' equivalent action potentials are generated to transmit information to external, or other bacteria, in the surrounding. The two systems are behaviorally similar, but quorum sensing in bacteria is more easily studied in depth than cell-cell signaling in eukaryotes [1]. Recent microbiology advances have determined that bacteria are an easily characterizable model organism with an extraordinarily complicated set of abilities, comparable cells or to the whole brain sensing. Among them is quorum sensing, a cell-cell signaling system that may have a common evolutionary origin with eukaryotic cell-cell signaling, and related to action potentials. Because of this comparative ease of study, bacterial dynamics are also more suited to direct interpretation than eukaryotic dynamics, i.e., those of the neuron.

The term action potential refers to the electrical signaling that occurs within neurons or bacteria biofilms, arising from changes in membrane potential from ion concentrations in the vicinity of membranes. An action potential is a rapid rise and subsequent fall in voltage, or membrane potential, across a cellular membrane with a characteristic pattern. Sufficient current is required to initiate a voltage response in a cell membrane; if the current is insufficient to depolarize the membrane to the threshold level, an action potential will not be triggered [2]. Membrane potential refers to the difference in charge between the inside and outside of a cell (such as neurons or bacteria), which is created due to the unequal distribution of ions on both sides of the cell.

The plasma membrane of a cell typically has a transmembrane potential of approximately $-100mV$ (negative inside) as a consequence of $k+$, $Na+$ and $Cl-$ concentration gradients that are maintained by active transport processes. Typically it is detected by means of optical techniques using biological markers [3]. For instance based on optogenetics by incorporating into an electrically excitable cell an optical reporter [4]. A signal is obtained from the optical reporter in response to a stimulation of the cell. In particular, potentiometric optical probes enable membrane potential measurements in organelles and in bacterial cells that are too small for microelectrodes. Potentiometric probes are an indirect method of detecting the translocation of these ions, whereas the fluorescent ion indicators can be used to directly measure changes in specific ion concentrations. Moreover, in conjunction with imaging techniques, these probes can be employed to map variations in membrane potential across excitable cells, with spatial resolution and sampling frequency that cannot be obtained using microelectrodes. Furthermore, patch-clamp technique allows direct measurements of electrical potential, but it is highly invasive. Patch-clamp technique has been applied only with isolated bacterial membranes or giant spheroplasts and protoplasts. Therefore, while patch clamp is a powerful technique for studying prokaryotic ion channels, it is not suitable for functional studies of membrane potential dynamics under physiological conditions.

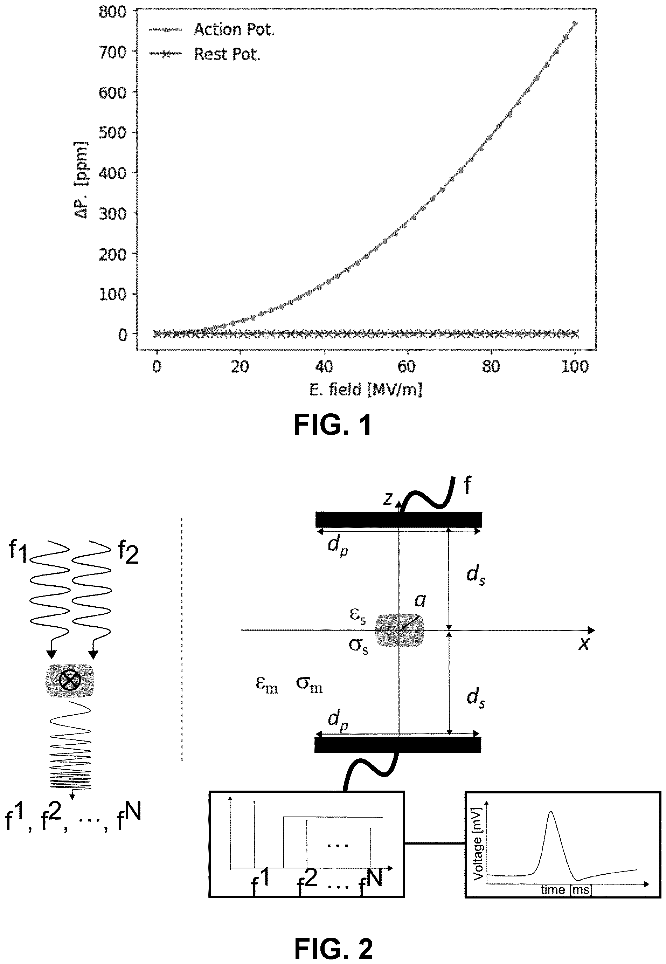

In many situations, electromagnetic waves interact with inhomogeneous particles and the corresponding electromagnetic-matter interaction process is referred to as scattering. A general scheme of the configuration of electromagnetic illumination and detection of scattering by a small bio-particle (e.g., bacteria) in a medium (e.g., water) is depicted in Fig. 2. Here, particle refers to a region in space characterized by a dielectric complex permittivity ($\varepsilon_s$, $\sigma_s$) which is different from that of the surrounding medium ($\varepsilon_m$, $\sigma_m$).

At microwave frequencies and for the typical dimensions of the experimental setups, the conditions are of near-field. In particular, the bio-particle is isolated due to using dilute enough solutions, only is considered single scattering (not considering multiple scattering) as described in [5]. But, in a general situation also considering non-dilute bio-particles (e.g., inside the brain), the same physical principles would apply, and multiple-scattering would be considered.

Scattering by a small spherical particle

When an electromagnetic wave impinges on an object, the incident wave is modeled as a plane electromagnetic wave (sufficiently accurate since the emitter is much larger than the particle). Assuming the electromagnetic wave to be polarized in the $z$ direction and propagating in the $x$ direction. Also assuming the particle to be a small spherical particle with permittivity $\varepsilon_s$ and radius $a$. Essentially, the particle sees a constant field as the plane wave impinges on it (and almost electrostatic field on the incident field). The incident field polarizes the particle making it look like an electric dipole. Since the incident field is a time harmonic, the small electric dipole will oscillate and radiate like a Hertzian dipole in the far field, but it can also be analyzed in the near-field. A Hertzian dipole can be approximated by a small current source so that $\vec{J}(\vec{r}) = \hat{z}Il\delta(\vec{r})$.

*Near-Field regime (a similar analysis could be performed for far-field regimes)*

The electric field is given by $\vec{E} = -j\omega\vec{A} - \nabla\Phi$, where the scalar potential term dominates over the vector potential in the near field of the scatterer. The scalar potential $\overrightarrow{\Phi(\vec{r})}$ is obtained from the Lorenz gauge as $\nabla \cdot \vec{A} = -j\omega\mu\epsilon\Phi$, resulting in

$$\Phi(\vec{r}) = \frac{-1}{j\omega\mu\epsilon}\nabla \cdot \vec{A} = \frac{-Il}{j\omega\epsilon 4\pi}\frac{\partial}{\partial z}\frac{1}{r}e^{-j\beta r}$$

. When close to the dipole, by assuming that $\beta r \ll 1$, a quasi-static approximation can be utilized for the potential (equivalent to ignoring retardation effect)

$$\frac{\partial}{\partial z}\frac{1}{r}e^{-j\beta r} \approx \frac{-\cos\theta}{r^2},$$

and reducing the potential to

$$\Phi(\vec{r}) \approx \frac{ql}{4\pi\epsilon r^2}\cos\theta.$$

This dipole induced in the small particle is formed in response to the incident field. The incident field can be approximated by a constant local static electric field $\vec{E}_{inc} = \hat{z}\,E_i$. The corresponding electrostatic potential for the incident field is then $\Phi_{inc} = -\hat{z}\,E_i$, so that $\vec{E}_{inc} \approx -\nabla\Phi_{inc} = \hat{z}E_i$. The scattered dipole potential from the spherical particle in the vicinity of it is given by

$$\Phi_{sca} = E_s\frac{a^3}{r^2}\cos\theta$$

. The electrostatic boundary problem is

$$E_s = \frac{\epsilon_s - \epsilon}{\epsilon_s + 2\epsilon}E_i$$

. As a result, the electric scattered field is given by

$$\vec{E}_{sca} = \left[\frac{k^2 a^3}{r}\frac{\epsilon_s - \epsilon}{\epsilon_s + 2\epsilon} - \frac{a^3}{r^3}\frac{\epsilon_s - \epsilon}{\epsilon_s + 2\epsilon}\right]E_i\,\hat{z} \approx -\frac{a^3}{r^3}\frac{\epsilon_s - \epsilon}{\epsilon_s + 2\epsilon}E_i\,\hat{z}$$

. A similar derivation can be obtained for the magnetic scattered near-field given by

$$\vec{H}_{sca} \approx \frac{k\,a^3}{r^2}\frac{\epsilon_s - \epsilon}{\epsilon_s + 2\epsilon}H_i\,\hat{z}$$

, where $\vec{H}_{inc} = \hat{z}\,H_i$ is the incident magnetic field.

Furthermore, in terms of electrical circuit equivalent representation of the scattered fields, the perturbed electrical field and magnetic fields results in a capacitance and inductance change between the electrodes. This change can be derived from the perturbed electric system energy caused by an induced dipole moment $\vec{p}$ as $\Delta U = \frac{1}{2}\Delta C\,V_i^2 = -\frac{1}{2}\vec{p} \cdot \vec{E}_i$, where $V_i$ is the driving voltage. Hence, the induced capacitance change can be expressed as

$$\Delta C = 4\pi a^3\Re\{\epsilon K_{CM}\}\frac{|E_i|^2}{V_i^2}.$$

The complex permittivity of biological particles with a membrane at rest, or when generating a potential, do not differ much from each state. The membrane's capacitance (related to the real part of the complex permittivity) of bacteria depends on its surface area, while the conductivity of the membrane depends on its cross-sectional area. Hence, it is not expected to easily observe a large variation between a biological particle at rest and at generating an action potential by making use of typical linear electromagnetic techniques, because these physical dimensions of the biological particles are not expected to have a major change.

Extracting information directly at the membrane of bio-particles with RF signals, would occur if the electrical conductivity or dielectric conductivity (in general, the complex permittivity) of a biological component varied significantly with the electric field intensity [6]. Symmetry arguments show that these higher-order terms should be zero in solids elements with inversion symmetry. In particular, dielectric polarization due to rotating dipoles, generally at microwave frequencies, a measurable nonlinearity due to saturation of rotation dipoles could be expected only at very low temperatures and intense field strengths, hence little nonlinearity expected. However, the only known example of a biological component that is detectably nonlinear for average extracellular tissue fields is a cell membrane. The neutral lipid bilayer separates regions of opposite polarity, so that the charge distribution is similar to that of a semiconductor junction. The nonlinearity was demonstrated by the observation that membranes rectified RF signals of frequencies below a few kHz. However, the value of the rectified signal fell rapidly at higher frequencies and at 100 kHz was a hundred times less than the low frequency value, and continues decreasing for higher frequencies.

Frequency harmonic generation from an interface can also result from the action of a static electric field on the bulk solution due to surface charges. This field is responsible for a $\chi^{(3)}$ susceptibility contribution to the SHG due to the polarization of solvent species by the surface charges. Therefore, the total second order polarization $P_{2w}$, including $\chi^{(2)}$ and $\chi^{(3)}$ contributions from a charged solid/liquid interface is given by $P_{2w} = \chi^{(2)}E_wE_w + \chi^{(3)}E_wE_w\Phi$. Fundamentally, the external very low frequency field (potential) $\Phi$ is provided by the inner membrane potential produced by the bio-particles of interest. When measurements of the non-linear effect are made by superposing a low intensity alternating field on a static field of high intensity, the quantity measured is the field-dependent incremental dielectric constant.

The theory of nonlinear dielectric effects is well documented, in particular in static fields [7]. Anomalous dielectric saturation effect is typically found in tertiary alcohol compounds, which have a relatively low value of the Kirkwood correlation factor, and for solutions of other alcohols in non-polar solvents or polar polymers, on the basis of the formation of molecular pairs in the liquid. The molecular pairs have a net dipole moment, depending on the angle between the molecules constituting the pair. The field influences the average dipole moment of the molecular pair in such a way that the high average dipole moment of the molecular pair is increased at higher field strengths.

Microwave non-thermal effects are postulated to result from a proposed direct interaction of the electric field with polar molecules in the reaction medium that is not related to a bulk temperature effect. Typically, in non-thermal microwave operation, the nonlinear microwave effects will be $\chi^{(2)} \approx 10^{-4}$ and $\chi^{(3)} \approx 10^{-9}$, while the membrane potential $\Phi \approx 10^8$ V/m and the incident field magnitude (non-thermal regime) $E_w = 1.5 \cdot 10^3$ V/m. The polarization will be dominated by the effect of the membrane potential, as shown in Fig. 1.

In the case of polar polymers, the origin of non-linear response is the saturation of arrangement of dipoles and its sign is negative. Furthermore, an electrical field potential due to the double layer as when a charged interface contacts an electrolyte solution, the ions in the solution will interact with the charged interface and distribute themselves accordingly. The presence of counterions screens the static electric field due to the charged interface and decreases the magnitude of the field as the distance from the interface increases. Phosphate buffer saline (PBS) is commonly applied as an electrolyte for biosensors application because ion concentration and osmolality of PBS are similar to the human body.

Proposes an experiment that tests whether the electric and magnetic structures of biological cells exhibit the nonlinear responses necessary for demodulation. A high Q cavity and very low noise amplification can be used to detect ultraweak nonlinear responses that appear as a second harmonic of a RF field incident on the sample. Nonlinear fields scattered from metabolically active biological cells grown in monolayer or suspended in medium can be distinguished from nonlinearities of the apparatus.

Estimates for the theoretical signal sensitivity and analysis of system noise indicate the possibility of detecting a microwave signal at 1.8 GHz (2nd harmonic of 900 MHz) as weak as one microwave photon per cell per second. The practical limit, set by degradation of the cavity Q, is extremely low compared to the much brighter thermal background, which has its peak in the infrared at a wavelength of about 17 $\mu$m and radiates 10 infrared photons per second per cell in the narrow frequency band within 0.5% of the peak. The system can be calibrated by introduction of known quantities of nonlinear material, e.g., a Schottky diode. Operating in the reverse frequency conversion direction for calibration purposes, for an input power of 160 $\mu$W at 900 MHz incident on such biological material, the apparatus is estimated to produce a robust output signal of 0.10 mV at 1.8 GHz if detected with a spectrum analyzer and a 30 dB gain low noise amplifier.

Discloses that the presence of harmonic products due to possible nonlinear interaction of amplitude modulated RF signals in living cells is best detected by using a cavity with high quality factor. Harmonic products generated by elementary oscillators can be trapped and accumulated in a cavity, permitting detection sensitivity much greater than in an open environment, where they would be radiated in all directions. The experimental method described in [9] is a systematic approach to detection of the non-Planck RF energy (if any) emitted by an exposed sample of living cells.

relates also to amplitude modulated RF signals and centered in a specific laboratory arrangement setup for the detection of RF signals with potentially high sensitivity. Hence, the authors discuss and elaborate on a potential detection system adding to the main signal carrier frequency (Microwave) a low frequency sideband signal (below RF) - based on a frequency downconversion process.

Description of the Invention

An object of the present invention is thus to provide a microwave sensing configuration for membrane potential measurement which overcomes the limitations of the prior art. For this purpose, the invention comprises the steps of exciting microwave oscillating potentials in order to produce mixing of the microwave illuminating electromagnetic field at given frequencies within the membrane of biological particles, which will generate a scattered field at the fundamental frequency and also at harmonics related to the non-linear relation between the voltage and current in the membrane of biological particles.

In particular, the frequency parametric process uses a Sum Frequency Generation from two input beams, which is an energy upconversion process. Typically, frequency downconversion processes, generally used in the literature, have efficiencies of 2 to 4 orders of magnitude less than frequency upconversion processes.

The above-cited object is fulfilled by a method with the characteristics of claim 1 and with a system with the characteristics of claim 8.

Embodiments of the present invention provide according to a first aspect a method for detecting microwave signals which carry information of the functional dynamics of biological particles. The method comprises illuminating one or more biological particles with microwave electromagnetic waves, the microwave electromagnetic waves being comprised in a non-thermal intensity regime, having frequencies with wavelengths much larger than characteristic dimensions of the biological particle(s) and being adapted to a functional dynamics signature of the biological particle(s); adapting/transforming the illuminating microwave electromagnetic waves to produce additional frequencies (i.e. harmonics) by an upconversion parametric process of frequency mixing in the vicinity region of the membrane of the biological particle(s) with a nonlinear relationship; capturing a response affected by the nonlinear relationship at different functional states of the biological particle(s); and detecting resulting frequency components of the captured response by means of a filtering element, so as to thereby infer the functional dynamics of the biological particle(s).

Particularly, the nonlinear relationship is a nonlinear current-voltage relationship producing a voltage potential at the membrane interface. The medium nonlinear relationship follows an anomalous dielectric saturation effect, which is typically found in tertiary alcohol compounds that have a relatively low value of the Kirkwood correlation factor, and for solutions of other alcohols in non-polar solvents or polar polymers, on the basis of the formation of molecular pairs in the liquid. Thus, in some embodiments, the microwave electromagnetic waves are adjusted/adapted to the medium comprising the biological particles(s), being said medium an alcohol, ionic biological buffer, a polymer, etc.

In an embodiment, the illuminating microwave electromagnetic waves are operated in power far below a threshold for thermal damaging the biological particle(s). Alternatively or complementarily, the illuminating microwave electromagnetic waves are operated such that the duration of the illumination is adapted to the temporal functional dynamics of the biological particle(s). Yet, alternatively or complementarily, the frequencies of the illuminating microwave electromagnetic waves can be adjusted to resonances of the dynamics of the biological particle(s).

In an embodiment, the method further comprises a step of isolating the frequencies of the illuminating microwave electromagnetic waves, either spatially or in frequency.

Embodiments of the present invention also provide according to a second aspect a system for detecting microwave signals which carry information of the functional dynamics of biological particles. The system comprises a biological particle, a microwave generator, an illumination adapter/controller, a microwave detector and a filter element.

The microwave generator is adapted/configured to produce microwave electromagnetic waves to illuminate the biological particle, the microwave electromagnetic waves being comprised in a non-thermal intensity regime, having frequencies with wavelengths much larger than characteristic dimensions of the biological particle and being adapted to a functional dynamics signature of the biological particle. The illumination adapter is adapted/configured to adapt the illuminating microwave electromagnetic waves to produce additional frequencies through an upconversion parametric process of frequency mixing in the vicinity region of the membrane of the biological particle with a nonlinear relationship. The microwave detector is adapted/configured to capture a response affected by the nonlinear relationship at different functional states of the biological particle. The filter element is adapted/configured to detect resulting frequency components of the captured response.

In an embodiment, the system also includes a spatial or frequency isolating element to isolate the frequencies of the illuminating microwave electromagnetic waves.

In an embodiment, the microwave generator is adapted to be pulsed and operated such that the duration of the illumination is adjusted to the temporal functional dynamics of the biological particle.

The illumination adapter and the microwave detector can be positioned either collinear or adjacent to each other. In other embodiments, the illumination adapter can be arranged at one of the sides of the biological particle whereas the microwave detector is arranged at another side of the biological particle. In any case, both elements/units do not need to be directly confronted.

In an embodiment, the filter element is a high-pass frequency filter. In another embodiment the filter element comprises a matched filter receiver configured to exploit matched filter signal processing schemes, for example Passive Intermodulation Products (PIMPs), among others.

In an embodiment, the microwave detector is based on sensitive quantum electromagnetic detection schemes.

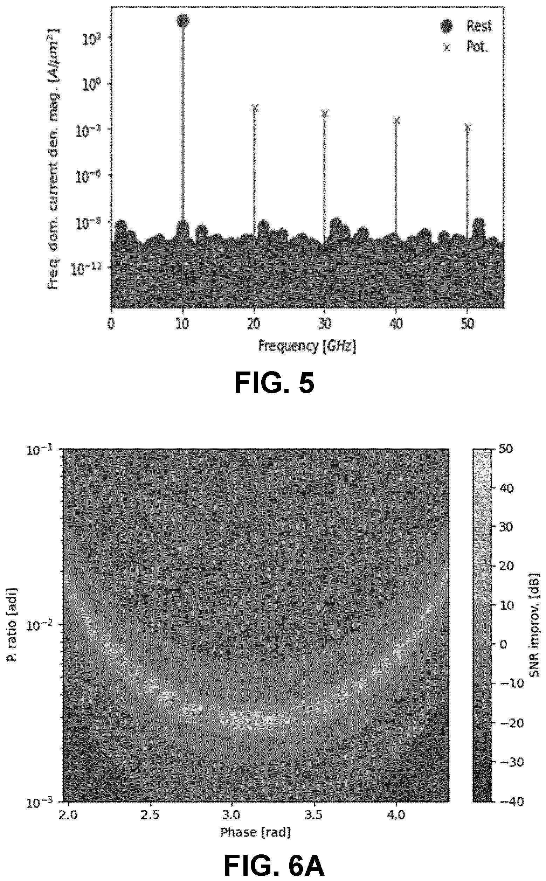

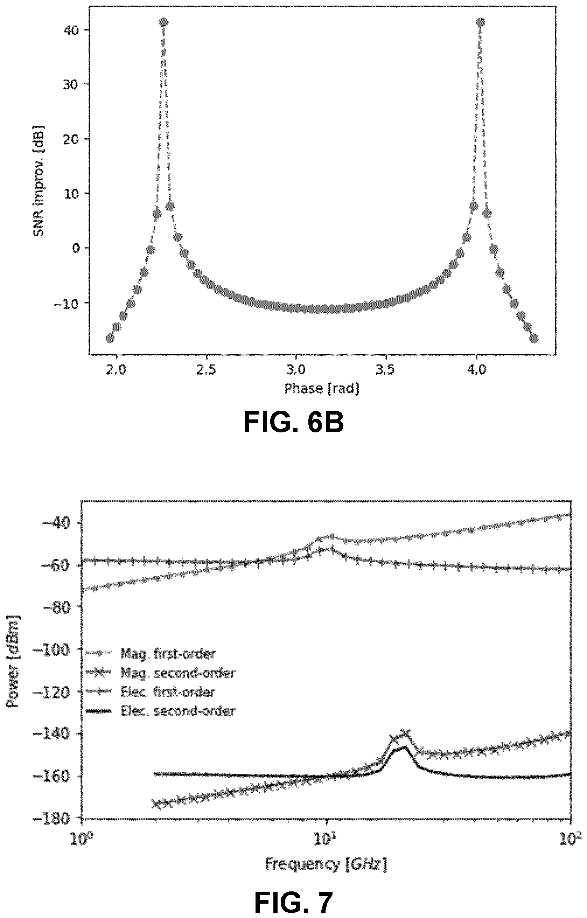

The present invention allows to measure minute signal differences that have limited the current state-of-the-art. In particular, the proposed method uses a system having two illumination frequencies (or more), at microwave frequencies. On one hand, these frequencies at least have to be separated more than the sidebands of the phase noise of the illumination microwave frequencies, while being compliant with the requirements explained next. On the other hand, non-linear intermodulation interference terms ($a$, $b$, $c$) in typical detection systems behave as $a A + b A^2 + c A^3$ with amplitude $A$, typically being the power $P = A^2/Z$, where $Z = 50$ is the typical impedance value. Considering that the amplitude $A$ consists of two (or more) sinusoidal amplitude signals with frequencies ($f_1$ and $f_2$), amplitudes ($A_1$ and $A_2$) and $\phi$ phase difference, equals to $A = A_1 \cos (2\pi f_1 t) + A_2 \cos (2\pi f_2 t + \phi)$. Considering the particular relation between the illumination frequencies $f_2 = 2f_1$ and working the generated signals through the system at frequency $f_1 + f_2$ corresponds to $0.75 c A_1^3 + b A_1 A_2 \cos \phi$. Notice that other relations between the chosen illumination frequencies and final observation frequency point also allow to perform the proposed method for reducing the nonlinear intermodulation interference. Hence, by suitably choosing the illumination frequencies, illumination power at each frequency and phase between them, an interference auto-cancellation (PIMPS technique) can be tailored to improve the signal level with respect to the intermodulation noise terms (as a signal-to-noise ratio improvement), as shown in Figs. 6A and 6B.

Brief Description of the Drawings

The previous and other advantages and features will be more fully understood from the following detailed description of embodiments, with reference to the attached figures, which must be considered in an illustrative and non-limiting manner, in which:

Fig. 1 illustrates the change in ppm in second harmonic power due to an action potential and rest potential. The static field strength in an action potential peaks at 100 MV/m, producing a change in second harmonic power of 800 ppm. While at rest potential no second harmonic change is produced.

Fig. 2 is a general scheme of the configuration of electromagnetic illumination and detection of scattering by a small bio-particle (e.g., bacteria) in a medium (e.g., water). The electromagnetic response of bacteria depends on their shape and size, their internal structure, and the electric conductivity and permittivity of the different bacterial cell components, which may depend on the bacterial physiological state but depend very little on the functional state of the bacteria. Scheme of the configuration and relevant values depicted, where $\varepsilon_s$ stands for permittivity of the cell, $\sigma_s$ conductivity, $a$ radius, $\varepsilon_m$ permittivity of the medium, $\sigma_m$ conductivity, $d_p$ is the length of the illumination and detection plates and $d_s$ distance to the cell (considered equally spaced)

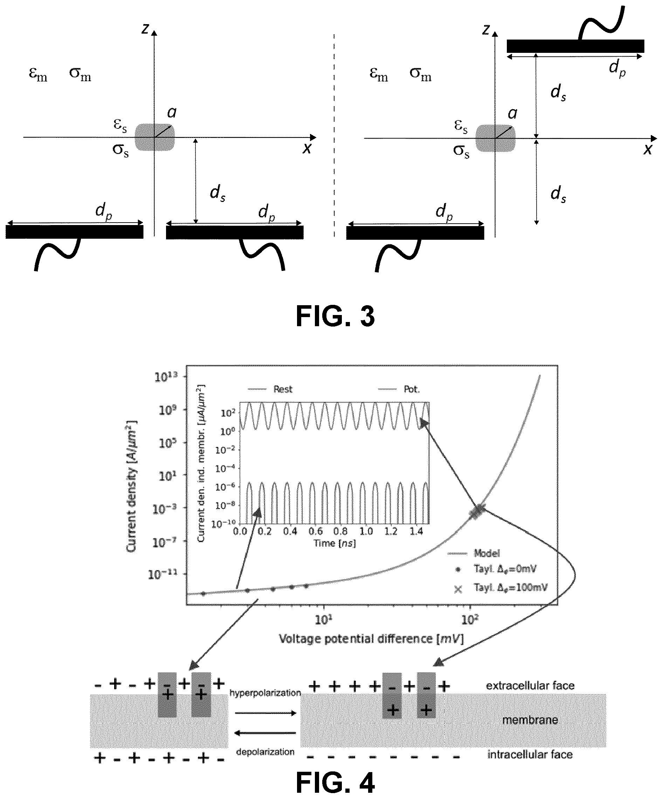

Fig. 3 shows different setups for putting into practice the process according to the invention, in which the detection element is positioned at different orientations with respect to the illumination element.

Fig. 4 illustrates the nonlinear voltage-current response of the membrane potential; (top) current density non-linear dependence with the differential voltage potential; (inset) induced current density due to an impinging microwave field at two different membrane potential states, while at rest and while at generating an action potential. The magnitudes of the induced current density differ in eight orders of magnitude; (bottom) shows a membrane scheme where fast-response operates by means of a change in their electronic structure, in response to a change in the surrounding electric field.

Fig. 5 illustrates the frequency domain analysis where the fundamental frequency (pump) scattered due to the bio-particle and its functional state is masked by the illumination itself, making it very difficult to sense the difference between the bacteria at rest and in an action potential state. Instead, the difference of the electromagnetic response of the bacteria in the two different functional states in the second frequency harmonic is around 8 orders of magnitude.

Figs. 6A and 6B shows the Passive Intermodulation Products auto-cancellation method. Consider a system's nonlinear intermodulation terms $a$, $b$ and $c$, with $a = 1$, $b = 10^{-3}$ and $c = 10^{-4}$, and -10 dBm impinging power for one of the frequencies. Fig. 6A plots the particular signal to noise ratio (SNR) improvement for specific power ratio and phase for the second illumination frequency. The SNR between the signal and the interference (due to the measurement equipment) are enlarged within specific power ratios and phase of the second illumination frequency with respect the first frequency. In particular, SNR improvements of more than 40 dB can be achieved, as shown in Fig. 6B.

Fig. 7 shows that the signal levels regarding magnetic fields are slightly higher compared to electric fields for higher frequencies. Considering the magnetic or electric field, the second-order signal level is close to the noise level. Different techniques can be applied in order to improve the sensitivity of detectors, such as novel quantum electro-magnetic sensing schemes.

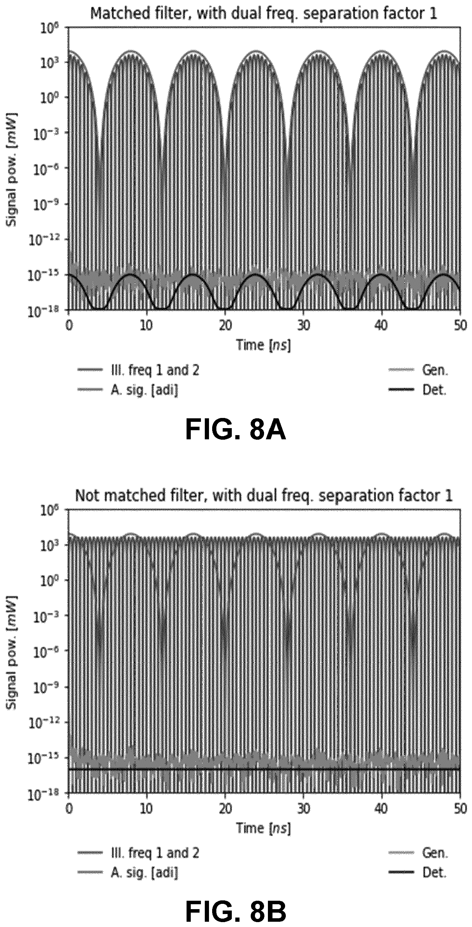

Figs. 8A and 8B show an example in which illumination with microwave signals is adapted to the signature of the functional dynamics of the biological particle, in order to detect these from the potential other signals present in the system and not carrying information of the functional dynamics of the biological particles.

Detailed Description of Preferred Embodiments

Present invention is based on the transformation of the illuminating energy in the microwave illuminating frequencies in order to produce subsequent harmonics within the nonlinear current-voltage dependence in the membranes of biological particles.

More in particular, present invention provides a method and system for measuring membrane potential which carries information on the functional dynamics of biological particles by means of a microwave nonlinear scattering. The invention operates microwave signals by means of the signal signature of the dynamics of the biological particles, combined with the non-linear response of the membrane of microorganisms, transforms the frequencies of the incident illuminating microwave electromagnetic field in a sequence of harmonics, and detects the resulting signal. The signal detected is a proxy for the biological particles functional dynamics.

According to a first embodiment, as shown in Fig. 2, this is achieved by illuminating the biological particle with microwave electromagnetic wave at frequencies $f_1$, $f_2$, . ... (e.g., 1 - 100 GHz), where the different illumination frequencies can be indicated as $f$, where the specific intensities of the illuminating microwave signals in the non-thermal regime (e.g., 0 - 30 dBm) and frequencies (e.g., with impinging frequencies much larger than the equivalent characteristic dimension of the biological particle of 1 $\mu$m - 1 mm, being $\Delta f$ in the order of 100 Hz - 10 GHz) are adjusted properly to produce the mixing of the frequencies, related by the principle of energy conservation $\Sigma f = \sum_{n=1}^{N} f^n$, within the membrane of the biological cell producing, with a relative high efficiency of transformation, several harmonics $f^1, f^2 \cdots, f^N$. Furthermore, higher order nonlinear terms become significant as voltage across the membrane of the biological particle is increased, such as in the process of generating an action potential.

Cellular as well as intracellular membranes exhibit a distinct nonlinear electrical behavior, due to the potential barrier resulting from the difference between inner and outer electrolytes and the action of ion-pumps. In the absence of an action potential, the transmembrane potential difference $\Delta_\phi$ is equal to the cell resting potential $V_0$ ($\approx$ -100 mV, in a typical cell). When an action potential occurs, a transmembrane excess potential appears $\Delta\phi = V_0 + \delta_\phi$. As a result, a transmembrane current density $\vec{J}_m$ flows. The current-voltage response is known to be fairly well approximated by a nonlinear diode-like relationship of the form $J_m = J_0(e^{\delta_\phi/V_T} - 1)$ with typical values $J_0 \approx 10^{-5}$ A/cm$^2$, $V_T \approx 5$ mV. Accordingly, all electrical variables of interest (e.g., field, currents, etc.) may be derived from the occurring single scalar potential $\Phi(\vec{r})$.

Referring back to Fig. 2, the cell's membrane is illuminated with microwave fields while the bias working point in the non-linear voltage-current model will be determined by the biological particle self-potential state. Depending on the state of the membrane potential, the adjusted impinging microwave signals will scatter having multiple generated frequency harmonics (due to the non-linear voltage-current model of the membrane), compared to the incident illuminating signals. In particular, using Taylor series expansion for the first two terms of the transmembrane current density $J_m$, the expected efficiency of the second-order process at $\delta_\phi$ = 100 mV, compared to $\delta_\phi$ = 0 mV, is 2 · 10$^8$ times larger, while the second-order process at $\delta_\phi$ = 100 mV compared to the first-order is 3 · 10$^{-5}$, which is still considerably efficient.

When impinging with microwave frequencies fields (e.g., 10 GHz), induced currents density is generated on the working point of the membrane potential state, where its magnitude and frequency harmonics are incremented when the cell is at an action potential compared to a rest state. The illumination frequencies (pump) scattering due to the membrane (around 10 nm) is obscured compared to the scattering generated by the cell's whole body (which is at least two orders of magnitude larger), making it very difficult to sense the difference between the bacteria at rest and in an action potential state. Instead, for instance for the second frequency harmonic there is around 11 orders of magnitude difference at the two cell's state, because it is essentially directly sensing the effect of the membrane potential and not the whole bacterial body. The expected relative second-order response at different frequencies, with respect to the fundamental pump frequency, at rest and at action potential state, are shown in Fig. 4. Furthermore, as known, the size of the bacteria scales the response with the cube of the radius, hence it is more challenging to detect 1 $\mu$m than 10 $\mu$m cells.

In Fig. 5 is shown the second harmonic generation signal level with respect the pump frequencies in the GHz range and the pump power in the mW range. It is shown, as it is common for frequency conversion, the non-linear relation between pump power and generated harmonics' power, while the dependence on pump frequency is smoother.

For typical room temperature and a bandwidth of 10 Hz, the noise level is around -164 dBm, while at 4 K cryogenic temperature levels the equivalent noise is around -182 dBm. Moreover, in the reactive near-field region of electromagnetic fields, the response levels of the electric and magnetic fields are not the same. In particular, the magnetic field, more closely related to currents, computes to have a higher level of electromagnetic response for higher frequencies, as shown in Fig. 7. Nevertheless, the expected intensity levels and the equivalent noise levels are of the same order. A cryogenic low noise amplifier, or other similar techniques, may be appropriate to experiment with to detect such low signal levels to achieve a reasonable SNR and enter into the sensitivity limits of detectors.

In Figs. 8A and 8B the illuminating microwave signals are adapted to the signature of the functional dynamics of the biological particle, in order to detect these from the potential other signals present in the system and not carrying information of the functional dynamics of the biological particles. Fig. 8A shows in the time domain the expected signal signature of the functional dynamics of the biological particles, the generated microwave signals adapted to the specific signature, the tailored generated signals from the functional dynamics of the biological particle and the detected signal carrying information of the functional dynamics of the biological particles. Fig. 8B shows in the time domain the expected signal signature of the functional dynamics of the biological particles, generated microwave signals not adapted to the specific signature, the generated signals from the functional dynamics of the biological particle and the not detected signal carrying information of the functional dynamics of the biological particles. Note that the signal signature is not scale, but not detrimental to the procedure, in order to easily show the steps described in this invention.

The scope of the present invention is defined in the following set of claims.

## References

[1] J. P. Stratford et al., "Electrically induced bacterial membrane-potential dynamics correspond to cellular proliferation capacity," PNAS, vol. 116, no. 19, pp. 9552-9557, May 2019, doi: 10.1073/pnas.1901788116.

[2] V. Pikov, X. Arakaki, M. Harrington, S. E. Fraser, and P. H. Siegel, "Modulation of neuronal activity and plasma membrane properties with low-power millimeter waves in organotypic cortical slices," J. Neural Eng., vol. 7, no. 4, p. 045003, Jul. 2010, doi: 10.1088/1741-2560/7/4/045003.

[3] "The Molecular Probes Handbook, 11th Edition." //www.thermofisher.com/es/es/home/references/molecular-probes-the-handbook/mp-handbook-download.html

[4] K. A. Deisseroth, E. A. Ferenczi, and P. Hegemann, "Devices, systems and methods for optogenetic modulation of action potentials in target cells," US20190125871A1, May 02, 2019 Accessed: Jan. 23, 2022. [Online]. Available: https://patents.google.com/patent/US20190125871A1/en?q=membrane+potential &oq=membrane+potential

[5] C. W. C., "Waves and fields in inhomogeneous media," CERN, Document Server, 1995. [Online]. Available: https://catalogue.library.cern/literature/32bs9-4xw77

[6] T. E. Khattab, "The Effect of High Power at Microwave Frequencies on the Linearity of Non-Polar Dielectrics in Space RF component," International Journal of Communications, Network and System Sciences, vol. 08, no. 02, Art. no. 02, Feb. 2015, doi: 10.4236/ijcns.2015.82002.

[7] R. Richert and W. Huang, "Time-resolved nonlinear dielectric responses in molecular systems," Journal of Non-Crystalline Solids, vol. 356, no. 11, pp. 787-793, Apr. 2010, doi: 10.1016/j.jnoncrysol.2009.08.047.

[8] Q. Balzano, "Proposed test for detection of nonlinear responses in biological preparations exposed to RF energy," Bioelectromagnetics, vol. 23, no. 4, pp. 278-287, 2002, doi: 10.1002/bem.10017.

[9] "RF nonlinear interactions in living cells-II: Detection methods for spectral signatures - Balzano - 2003 - Bioelectromagnetics - Wiley Online Library." https://onlinelibrary.wiley.com/doi/10.1002/bem.10125 (accessed Aug. 12, 2021).

[10] Q. Balzano, V. Hodzic, R. W. Gammon, and C. C. Davis, "A doubly resonant cavity for detection of RF demodulation by living cells," Bioelectromagnetics, vol. 29, no. 2, pp. 81-91, 2008, doi: 10.1002/bem.20365.

## Claims

1. A method for detecting microwave signals which carry information of the functional dynamics of biological particles, the method comprising the steps of:

   a) illuminating at least one biological particle with microwave electromagnetic waves, the microwave electromagnetic waves being comprised in a non-thermal intensity regime, having frequencies with wavelengths much larger than characteristic dimensions of the at least one biological particle and being adapted to a functional dynamics signature of the biological particle;

   b) adapting the illuminating microwave electromagnetic waves to produce additional frequencies by an upconversion parametric process of frequency mixing in a region of vicinity of a membrane of the biological particle with a nonlinear relationship;

   c) capturing a response affected by the nonlinear relationship at different functional states of the biological particle; and

   d) detecting resulting frequency components of the captured response by means of a filtering element,

   so as to thereby infer the functional dynamics of the biological particle.

2. The method according to claim 1, further comprising adapting or adjusting the microwave electromagnetic waves to a given medium containing the at least one biological particle, said given medium including an alcohol, an ionic biological buffer or a fluid polymer.

3. The method according to claim 1 or 2, wherein the nonlinear relationship comprises a nonlinear current-voltage relationship producing a voltage potential at an interface of the membrane of the biological particle.

4. The method according to any one of claims 1-3, wherein the illuminating microwave electromagnetic waves are operated in power far below a threshold for thermal damaging the biological particle.

5. The method according to any one of claims 1-3, wherein the illuminating microwave electromagnetic waves are operated such that a duration of the illumination is adapted to the temporal functional dynamics of the biological particle.

6. The method according to any one of claims 1-3, wherein the frequencies of the illuminating microwave electromagnetic waves are adjusted to resonances of the dynamics of the biological particle.

7. The method according to any one of the previous claims, further comprising a step of isolating the fundamental frequencies of the illuminating microwave electromagnetic waves, either spatially or in frequency.

**8.** A system for detecting microwave signals which carry information of the functional dynamics of biological particles, comprising:

at least one biological particle;
a microwave generator configured to produce microwave electromagnetic waves to illuminate at least one biological particle, the microwave electromagnetic waves being comprised in a non-thermal intensity regime, having frequencies with wavelengths much larger than characteristic dimensions of the at least one biological particle and being adapted to a functional dynamics signature of the biological particle;
an illumination adapter configured to adapt the illuminating microwave electromagnetic waves to produce additional frequencies through an upconversion parametric process of frequency mixing in a region of vicinity of a membrane of the biological particle with a nonlinear relationship;
a microwave detector configured to capture a response affected by the nonlinear relationship at different functional states of the biological particle; and
a filter element configured to detect resulting frequency components of the captured response.

**9.** The system according to claim 8, further comprising a spatial or frequency isolating element configured to isolate the fundamental frequencies of the illuminating microwave electromagnetic waves.

**10.** The system according to claim 8 or 9, wherein the microwave generator is adapted to be pulsed and operated such that a duration of the illumination is adjusted to the temporal functional dynamics of the biological particle.

**11.** The system according to any one of the previous claims 8 to 10, wherein the illumination adapter and the microwave detector are positioned either collinear or adjacent to each other, or each one at a side of the biological particle.

**12.** The system according to any one of the previous claims 8 to 11, wherein the filter element is a high-pass frequency filter.

**13.** The system according to any one of the previous claims 8 to 11, wherein the filter element comprises a matched filter receiver configured to exploit signal processing schemes.

**14.** The system according to claim 13, wherein the signal processing schemes are based on Passive Intermodulation Products interferometric cancellation.

**15.** The system according to any one of the previous claims 8 to 14, wherein the microwave detector is based on sensitive quantum electromagnetic detection schemes.

**Patentansprüche**

**1.** Verfahren zum Detektieren von Mikrowellensignalen, die Informationen über die funktionelle Dynamik von biologischen Teilchen tragen, wobei das Verfahren die folgenden Schritte umfasst:

a) Beleuchten mindestens eines biologischen Teilchens mit elektromagnetischen Wellen im Mikrowellenbereich, wobei die elektromagnetischen Wellen im Mikrowellenbereich in einem nicht-thermischen Intensitätsregime umfasst sind, Frequenzen mit Wellenlängen aufweisen, die viel größer als die charakteristischen Abmessungen des mindestens einen biologischen Teilchens sind, und an eine Signatur der funktionalen Dynamik des biologischen Teilchens angepasst sind;
b) Anpassen der beleuchtenden elektromagnetischen Wellen im Mikrowellenbereich, um zusätzliche Frequenzen durch einen parametrischen Aufwärtskonversionsprozess der Frequenzmischung in einem Bereich in der Nähe einer Membran des biologischen Teilchens mit einer nichtlinearen Beziehung herzustellen;
c) Erfassen einer Reaktion, die durch die nichtlineare Beziehung beeinflusst wird, bei unterschiedlichen Funktionszuständen des biologischen Teilchens; und
d) Detektieren resultierender Frequenzkomponenten der erfassten Reaktion mit Hilfe eines Filterelements,

um dadurch auf die funktionelle Dynamik des biologischen Teilchens zu schließen.

**2.** Verfahren nach Anspruch 1, ferner umfassend das Anpassen oder Einstellen der elektromagnetischen Wellen im Mikrowellenbereich an ein gegebenes Medium, das das mindestens eine biologische Teilchen enthält, wobei das gegebene Medium einen Alkohol, einen ionischen biologischen Puffer oder ein Fluidpolymer einschließt.

**3.** Verfahren nach Anspruch 1 oder 2, wobei die nichtlineare Beziehung eine nichtlineare Strom-Spannungs-Beziehung umfasst, die ein Spannungspotenzial an einer Schnittstelle der Membran des biologischen Teilchens herstellt.

**4.** Verfahren nach einem der Ansprüche 1-3, wobei die beleuchtenden elektromagnetischen Wellen im Mikrowellenbereich mit einer Leistung betrieben werden, die weit unter einem Schwellenwert für die thermische Schädigung des biologischen Teilchens liegt.

**5.** Verfahren nach einem der Ansprüche 1-3, wobei die beleuchtenden elektromagnetischen Wellen im Mikrowellenbereich derart betrieben werden, dass eine Dauer der Beleuchtung an die zeitliche funktionale Dynamik des biologischen Teilchens angepasst ist.

**6.** Verfahren nach einem der Ansprüche 1-3, wobei die Frequenzen der beleuchtenden elektromagnetischen Wellen im Mikrowellenbereich auf Resonanzen der Dynamik des biologischen Teilchens eingestellt sind.

**7.** Verfahren nach einem der vorhergehenden Ansprüche, ferner umfassend einen Schritt des Isolierens der Grundfrequenzen der beleuchtenden elektromagnetischen Wellen im Mikrowellenbereich, entweder räumlich oder frequenzmäßig.

**8.** System zum Detektieren von Mikrowellensignalen, die Informationen über die funktionelle Dynamik von biologischen Teilchen tragen, Folgendes umfassend:

mindestens ein biologisches Teilchen;
einen Mikrowellengenerator, der dazu konfiguriert ist, elektromagnetische Wellen im Mikrowellenbereich herzustellen, um mindestens ein biologisches Teilchen zu beleuchten, wobei die elektromagnetischen Wellen im Mikrowellenbereich in einem nicht-thermischen Intensitätsregime umfasst sind, Frequenzen mit Wellenlängen aufweisen, die viel größer als die charakteristischen Abmessungen des mindestens einen biologischen Teilchens sind, und an eine Signatur der funktionalen Dynamik des biologischen Teilchens angepasst sind;
einen Beleuchtungsadapter, der dazu konfiguriert ist, die beleuchtenden elektromagnetischen Wellen im Mikrowellenbereich anzupassen, um zusätzliche Frequenzen über einen parametrischen Aufwärtskonversionsprozess der Frequenzmischung in einem Bereich in der Nähe einer Membran des biologischen Teilchens mit einer nichtlinearen Beziehung herzustellen;
einen Mikrowellendetektor, der dazu konfiguriert ist, eine Reaktion zu erfassen, die durch die nichtlineare Beziehung beeinflusst wird, bei unterschiedlichen Funktionszuständen des biologischen Teilchens; und
ein Filterelement, das dazu konfiguriert ist, resultierende Frequenzkomponenten der erfassten Reaktion zu detektieren.

**9.** System nach Anspruch 8, ferner umfassend ein Raum- oder Frequenzisolierelement, das dazu konfiguriert ist, die Grundfrequenzen der beleuchtenden elektromagnetischen Wellen im Mikrowellenbereich zu isolieren.

**10.** System nach Anspruch 8 oder 9, wobei der Mikrowellengenerator dazu angepasst ist, gepulst zu werden und derart betrieben zu werden, dass eine Dauer der Beleuchtung an die zeitliche funktionale Dynamik des biologischen Teilchens eingestellt ist.

**11.** System nach einem der vorhergehenden Ansprüche 8 bis 10, wobei der Beleuchtungsadapter und der Mikrowellendetektor entweder kollinear oder benachbart zueinander oder jeweils an einer Seite des biologischen Teilchens positioniert sind.

**12.** System nach einem der vorhergehenden Ansprüche 8 bis 11, wobei das Filterelement ein Hochpassfrequenzfilter ist.

**13.** System nach einem der vorhergehenden Ansprüche 8 bis 11, wobei das Filterelement einen übereinstimmenden Filterempfänger umfasst, der dazu konfiguriert ist, Signalverarbeitungsschemata auszunutzen.

**14.** System nach Anspruch 13, wobei die Signalverarbeitungsschemata auf der interferometrischen Auslöschung passiver Intermodulationsprodukte basieren.

**15.** System nach einem der vorhergehenden Ansprüche 8 bis 14, wobei der Mikrowellendetektor auf sensitiven quantenelektromagnetischen Detektionsschemata basiert.

**Revendications**

**1.** Procédé de détection de signaux micro-ondes portant des informations de la dynamique fonctionnelle des particules biologiques, comprenant les étapes suivantes :

a) éclairer au moins une particule biologique avec des ondes électromagnétiques à micro-ondes, les ondes électromagnétiques à micro-ondes étant comprises dans un régime d'intensité non thermique, ayant des fréquences avec des longueurs d'onde beaucoup plus grandes que les dimensions caractéristiques de l'au moins une particule biologique et étant adaptées à une signature de dynamique fonctionnelle de la particule biologique ;
b) adapter les ondes électromagnétiques à micro-ondes éclairantes pour produire des fréquences supplémentaires par un processus paramétrique de conversion ascendante de mélange de fréquences dans une région à proximité d'une membrane de la particule biologique avec une relation non linéaire ;

c) capter une réponse affectée par la relation non linéaire à différents états fonctionnels de la particule biologique ; et

d) détecter des composantes de fréquence résultantes de la réponse captée au moyen d'un élément filtrant,

afin d'en déduire la dynamique fonctionnelle de la particule biologique.

**2.** Procédé selon la revendication 1, comprenant en outre l'adaptation ou l'ajustement des ondes électromagnétiques à micro-ondes à un milieu donné contenant l'au moins une particule biologique, ledit milieu donné comprenant un alcool, un tampon biologique ionique ou un polymère fluide.

**3.** Procédé selon la revendication 1 ou 2, dans lequel la relation non linéaire comprend une relation courant-tension non linéaire produisant un potentiel de tension à une interface de la membrane de la particule biologique.

**4.** Procédé selon l'une quelconque des revendications 1-3, dans lequel les ondes électromagnétiques à micro-ondes éclairantes sont utilisées à une puissance bien inférieure à un seuil de dommage thermique de la particule biologique.

**5.** Procédé selon l'une quelconque des revendications 1-3, dans lequel les ondes électromagnétiques à micro-ondes éclairantes sont utilisées de telle sorte que la durée de l'éclairage est adaptée à la dynamique fonctionnelle temporelle de la particule biologique.

**6.** Procédé selon l'une quelconque des revendications 1-3, dans lequel les fréquences des ondes électromagnétiques à micro-ondes éclairantes sont ajustées aux résonances de la dynamique de la particule biologique.

**7.** Procédé selon l'une quelconque des revendications précédentes, comprenant en outre une étape consistant à isoler les fréquences fondamentales des ondes électromagnétiques à micro-ondes éclairantes, soit dans l'espace soit dans la fréquence.

**8.** Système de détection des signaux micro-ondes portant des informations de la dynamique fonctionnelle des particules biologiques, comprenant :

au moins une particule biologique ;

un générateur de micro-ondes configuré pour produire des ondes électromagnétiques à micro-ondes afin d'éclairer au moins une particule biologique, les ondes électromagnétiques à micro-ondes étant comprises dans un régime d'intensité non thermique, ayant des fréquences avec des longueurs d'onde beaucoup plus grandes que les dimensions caractéristiques de l'au moins une particule biologique et étant adaptées à une signature de dynamique fonctionnelle de la particule biologique ;

un adaptateur d'éclairage configuré pour adapter les ondes électromagnétiques à micro-ondes éclairantes afin de produire des fréquences supplémentaires par un processus paramétrique de conversion ascendante de mélange de fréquences dans une région à proximité d'une membrane de la particule biologique avec une relation non linéaire ;

un détecteur de micro-ondes configuré pour capter une réponse affectée par la relation non linéaire à différents états fonctionnels de la particule biologique ; et

un élément filtrant configuré pour détecter des composantes de fréquence résultantes de la réponse captée.

**9.** Système selon la revendication 8, comprenant en outre un élément d'isolation spatiale ou de fréquence configuré pour isoler les fréquences fondamentales des ondes électromagnétiques à micro-ondes éclairantes.

**10.** Système selon la revendication 8 ou 9, dans lequel le générateur de micro-ondes est adapté pour être pulsé et utilisé de telle sorte que la durée de l'éclairage est adaptée à la dynamique fonctionnelle temporelle de la particule biologique.

**11.** Système selon l'une quelconque des revendications précédentes 8 à 10, dans lequel l'adaptateur d'éclairage et le détecteur de micro-ondes sont positionnés soit de manière colinéaire ou adjacente l'un à l'autre, soit chacun d'un côté de la particule biologique.

**12.** Système selon l'une quelconque des revendications précédentes 8 à 11, dans lequel l'élément filtrant est un filtre de fréquence passe-haut.

**13.** Système selon l'une quelconque des revendications précédentes 8 à 11, dans lequel l'élément filtrant comprend un récepteur à filtre adapté configuré pour exploiter des schémas de traitement du signal.

**14.** Système selon la revendication 13, dans lequel les schémas de traitement du signal sont basés sur l'annulation interférométrique des produits d'intermodulation passifs.

**15.** Système selon l'une quelconque des revendications précédentes 8 à 14, dans lequel le détecteur de micro-ondes est basé sur des schémas de détection électromagnétique quantique sensibles.

**FIG. 1**

**FIG. 2**

**FIG. 3**

**FIG. 4**

FIG. 5

FIG. 6A

## FIG. 6B

## FIG. 7

FIG. 8A

FIG. 8B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20190125871 A1, K. A. Deisseroth, E. A. Ferenczi, and P. Hegemann

### Non-patent literature cited in the description

- **J. P. STRATFORD et al.** Electrically induced bacterial membrane-potential dynamics correspond to cellular proliferation capacity. *PNAS,* May 2019, vol. 116 (19), 9552-9557
- **V. PIKOV ; X. ARAKAKI ; M. HARRINGTON ; S. E. FRASER ; P. H. SIEGEL.** Modulation of neuronal activity and plasma membrane properties with low-power millimeter waves in organotypic cortical slices. *J. Neural Eng.,* July 2010, vol. 7 (4), 045003
- The Molecular Probes Handbook
- **C. W. C.** Waves and fields in inhomogeneous media. *CERN, Document Server,* 1995, https://catalogue.library.cern/literature/32bs9-4xw77
- **T. E. KHATTAB.** The Effect of High Power at Microwave Frequencies on the Linearity of Non-Polar Dielectrics in Space RF component. *International Journal of Communications, Network and System Sciences,* February 2015, vol. 08 (02
- **R. RICHERT ; W. HUANG.** Time-resolved non-linear dielectric responses in molecular systems. *Journal of Non-Crystalline Solids,* April 2010, vol. 356 (11), 787-793
- **Q. BALZANO.** Proposed test for detection of nonlinear responses in biological preparations exposed to RF energy. *Bioelectromagnetics,* 2002, vol. 23 (4), 278-287
- *RF nonlinear interactions in living cells-II: Detection methods for spectral signatures - Balzano - 2003 - Bioelectromagnetics - Wiley Online Library.,* 12 August 2021, https://onlinelibrary.wiley.com/doi/10.1002/bem.10125
- **Q. BALZANO ; V. HODZIC ; R. W. GAMMON ; C. C. DAVIS.** A doubly resonant cavity for detection of RF demodulation by living cells. *Bioelectromagnetics,* 2008, vol. 29 (2), 81-91